# EUROPEAN PATENT SPECIFICATION

(11) **EP 1 478 376 B1**
(45) Date of publication and mention of the grant of the patent: **08.09.2010**
(21) Application number: 02727769.8
(22) Date of filing: 31.05.2002
(51) Int. Cl.: A61K 35/74, A61P 37/04

(54) **IMMUNOSTIMULATORY AGENT COMPRISING A BIOMASS OF METHANOTROPHIC BACTERIUM**
Immunstimulierendes Mittel enthaltend eine Biomasse von methanotrophem Bakterium
BIOMASSE COMPRENANT UNE BACTERIE METHANOTROPHIQUE COMME AGENT IMMUNOSTIMULANT

(30) Priority: 28.02.2002 GB 0204722
(43) Date of publication of application: 24.11.2004
(73) Proprietor: Statoil ASA, 4035 Stavanger (NO)
(72) Inventor: JORGENSEN, Lars, Norferm Danmark AS, 5230 Odense (DK); JOHANNESSEN, Arild, N-4326 Sandnes (NO); JENSEN, Karen Moller, Norferm Danmark AS, 5230 Odense (DK); KLEPPE, Gunnar, N-4044 Hafrsfjord (NO)
(74) Representative: Golding, Louise Ann
(86) International application number: PCT/GB2002/002555
(87) International publication number: WO 2003/072133

(56) References cited:
- WO-A-01/60974
- WO-A-03/015534
- US-A- 5 314 820
- DAHLGREN U I ET AL: "Expression of a dietary protein in E. coli renders it strongly antigenic to gut lymphoid tissue." IMMUNOLOGY. ENGLAND AUG 1991, vol. 73, no. 4, August 1991 (1991-08), pages 394-397, XP008017321 ISSN: 0019-2805
- KLEPPE G: "BIOPROTEIN, A NEW HIGH QUALITY SINGLE CELL PROTEIN BASED ON NATURAL GAS" NUTRECO AQUACULTURE BUSINESS CONFERENCE, XX, XX, 13 May 1998 (1998-05-13), pages 75-78, XP008010744
- MOLCK, ANNE-MARIE (1) ET AL: "Immunotoxicity of nucleic acid reduced BioProtein: A bacterial derived single cell protein: In Wistar rats." TOXICOLOGY, (JUNE 5TH, 2002) VOL. 174, NO. 3, PP. 183-200. HTTP://WWW.ELSEVIER.COM/LOCATE/TOXICOL. PRINT. , XP002241540
- STEPANKOVA R ET AL: "Prolonged survival of AVN Wistar rats with transplanted Yoshida sarcoma and increase of granular lymphocytes after administration of Bacillus firmus and their crude lipids." FOLIA MICROBIOLOGICA, (1995) 40 (4) 413-6. , XP008016556

## Description

The present invention relates generally to enhancement of the immune response in human and non-human animals, e.g. immunostimulation. More specifically, the invention relates to the use of a biomass derived from a methanotrophic bacterium-containing culture as an immunostimulant, e.g. to increase the resistance to infection of a human or non-human animal.

The role of the immune system in any animal is crucial for fighting off infection, for example in the control of diseases caused by external agents such as viruses, bacteria and other pathogens.

There is currently much interest in the use of agents which are able to produce immunostimulatory effects in both humans and non-human animals and which are thus able to maintain and/or enhance the response of the immune system. In this way, the ability of an animal to fight off infection is increased resulting in overall enhanced health and vitality.

It is known that certain bacteria, and extracts thereof, when given orally to animals can enhance the immune response to certain antigens. For example, enhanced immune response caused by certain bacterial peptidoglycans has been reported. Various yeasts such as those belonging to the genus *Saccaromyces cerevisiae* and their extracts, e.g. their insoluble glucans, have also been found to exhibit non-specific immunostimulation properties. However, there remains a a need for alternative materials which are capable of stimulating (e.g. enhancing) the immune response, especially materials which also exhibit good nutritional properties.

The present invention provides an immunostimulatory agent which is derived from a microbial culture containing a methanotrophic bacterium, preferably from a microbial culture containing the bacterium *Methylococcus capsulatus*, in addition to *Ralstonia sp*., *Brevibacillus agri* and *Aneurinibacillus sp.*

Thus, according to one aspect, the present invention provides a composition comprising a biomass obtained from a methanotrophic-bacterium containing culture for use as an immunostimulatory agent, e.g. for use in a medicament, e.g for use as maintaining and/or enhancing the immune system of a human or non-human animal body.

According to a further aspect the invention provides the use of a composition as herein described in the manufacture of a medicament for use as an immunostimulant, e.g. for use in maintaining and/or enhancing the immune system of a human or non-human animal body.

As used herein, the term "immunostimulatory" refers to enhanced function of cells involved in normal immune responses. Thus, enhanced cellular and/or humoral responses mediated by lymphocytes in the animal are observed. Enhanced responses may be reflected by increased complement activity, lysozyme activity, cellular immune responses or immunoglobulin levels as described hereinafter. Conveniently, immunoglobulin levels, e.g. IgG levels, are examined in an appropriate sample and compared against such levels in a corresponding sample from a normal human or non-human animal, i.e. a human or non-human animal to which the immunostimulant in accordance with the invention has not been administered. Immunoglobulin levels for a particular immunoglobulin type (e.g. IgG or IgA) or specific to one or more specific antigens may be examined. An enhanced function is reflected by a statistically significant increase (e.g. greater than about 5%, preferably 10%) in one or more of the above, or other appropriate indicators of the level of immune response.

The biomass for use according to the invention is derived from a microbial culture which comprises a methanotrophic bacterium, optionally in combination with one or more species of heterotrophic bacteria, especially preferably a combination of methanotrophic and heterotrophic bacteria. As used herein, the term "methanotrophic" encompasses any bacterium which utilizes methane, methanol or formaldehyde for growth. The term "heterotrophic" is used for bacteria that utilize organic substrates other than methane, methanol or formaldehyde for growth.

The bacterial biomass for use as herein described may be formed by growth of the bacteria on a suitable medium or substrate. The exact nature of the growth medium used to produce the biomass is not critical and a variety of suitable substrates may be used.

Conveniently, the biomass may be produced by a fermentation process in which oxygen and a suitable substrate such as a liquid or gaseous hydrocarbon, an alcohol or carbohydrate, e.g. methane, methanol or natural gas, together with a nutrient mineral solution are fed to a tubular reactor containing the microorganisms. A number of such processes are well known and described in the art, for example in WO 01/60974, DK-B-170824, EP-A-418187 and EP-A-306466.

The biomass material for use as herein described will preferably be derived from fermentation on hydrocarbon fractions or on natural gas. Especially preferred are biomass materials derived from the fermentation of natural gas. Generally in such processes, as the concentration of microorganisms increases within the fermentor, a portion of the reactor contents or broth is withdrawn and the microorganisms may be separated by techniques well known in the art, e.g. centrifugation and/or ultrafiltration. Conveniently, in such a fermentation process, the broth will be continuously withdrawn from the fermentor and will have a cell concentration between 1 and 5% by weight, e.g. about 3% by weight.

Preferred bacteria for use according to the invention include *Methylococcus capsulatus* (Bath), a thermophilic bacterium originally isolated from the hot springs in Bath, England and deposited as NCIMB 11132 at The National Collections of Industrial and Marine Bacteria, Aberdeen, Scotland. *M*. *capsulatus* (Bath) has optimum growth at about 45°C, although growth can occur between 37°C and 52°C. It is a gram-negative, non-motile spherical cell, usually occurring in pairs. The intracellular membranes are arranged as bundles of vesicular discs characteristic of Type I methanotrophs. *M. capsulatus* (Bath) is genetically a very stable organism without known plasmids. It can utilize methane or methanol for growth and ammonia, nitrate or molecular nitrogen as a source of nitrogen for protein synthesis.

Other bacteria suitable for use according to the invention include the heterotrophic bacteria DB3, strain NCIMB 13287 (*Ralstonia sp*. formerly known as *Alcaligenes acidovorans* DB3), DB5, strain NCIMB 13289 (*Brevibacillus agri* formerly known as *Bacillus firmus* DB5) and DB4, strain NCIMB 13288 (*Aneurinibacillus sp.* formerly known as *Bacillus* brevis DB4) which each have optimum growth at a temperature of about 45°C.

DB3 is a gram-negative, aerobic, motile rod belonging to the genus *Ralstonia* which can use ethanol, acetate, propionate and butyrate for growth. DB4 is a gram-positive, endospore-forming, aerobic rod belonging to the genus *Aneurinibacillus* which can utilize acetate, D-fructose, D-mannose, ribose and D-tagatose. DB5 is a gram-positive, endospore-forming, motile, aerobic rod of the genus *Brevibacillus* which can utilize acetate, N-acetyl-glucosamine, citrate, gluconate, D-glucose, glycerol and mannitol.

One example of a fermentation process which uses natural gas as the sole carbon and energy source is that described in EP-A-306466 (Dansk Bioprotein). This process is based on the continuous fermentation of the methanotropic bacterium *M*. *capsulatus* grown on methane. Air or pure oxygen is used for oxygenation and ammonia is used as the nitrogen source. In addition to these substrates, the bacterial culture will typically require water, phosphate (e.g. as phosphoric acid) and several minerals which may include magnesium, calcium, potassium, iron, copper, zinc, manganese, nickel, cobalt and molybdenum, typically used as sulphates, chlorides or nitrates.

Natural gas mainly consists of methane, although its composition will vary for different gas fields. Typically, natural gas may be expected to contain about 90% methane, about 5% ethane, about 2% propane and some higher hydrocarbons. During the fermentation of natural gas, methane is oxidized by methanotrophic bacteria to biomass and carbon dioxide. Methanol, formaldehyde and formic acid are metabolic intermediates. Formaldehyde and to some extent carbon dioxide are assimilated into biomass. However, methanotrophic bacteria are unable to use substrates comprising carbon-carbon bonds for growth and the remaining components of natural gas, i.e. ethane, propane and to some extent higher hydrocarbons, are oxidized by methanotrophic bacteria to produce the corresponding carboxylic acids (e.g. ethane is oxidized to acetic acid). Such products can be inhibitory to methanotrophic bacteria and it is therefore important that their concentrations remain low, preferably below 50 mg/1, during the production of the biomass. This problem can be addressed by the combined use of one or more heterotrophic bacteria which are able to utilize the metabolites produced by the methanotrophic bacteria. Such bacteria are also capable of utilizing organic material released to the fermentation broth by cell lysis. This is important in order to avoid foam formation and also serves to minimize the risk of the culture being contaminated with undesirable bacteria. A combination of methanotrophic and heterotrophic bacteria results in a stable and high yielding culture and is particularly preferred for use according to the invention.

During production of the biomass, the pH of the fermentation mixture will generally be regulated to between about 6 and 7, e.g. to 6.5 ± 0.3. Suitable acids/bases for pH regulation may be readily selected by those skilled in the art. Particularly suitable for use in this regard are sodium hydroxide and sulphuric acid. During fermentation the temperature within the fermentor should preferably be maintained to within the range of from 40°C to 50°C, most preferably 45°C ± 2°C.

Especially preferred for use in accordance with the invention is a microbial culture comprising a combination of the methanotrophic bacterium *Methylococcus capsulates* (Bath) (strain NCIMB 11132), and the heterotrophic bacteria DB3 (strain NCIMB 13287) and DB 5 (strain NCIMB 13289), optionally in combination with DB4 (strain NCIMB 13288). The role of DB3 is to utilize acetate and propionate produced by *M*. *capsulatus* (Bath) from ethane and propane in the natural gas. DB3 may account for up to 10%, e.g. about 6 to 8%, of the total cell count of the resulting biomass. The role of DB4 and DB5 is to utilize lysis products and metabolites in the medium. Typically, DB4 and DB5 will each account for less than 1% of the cell count during continuous fermentation.

Suitable fermentors for use in preparing the biomass are those of the loop-type, such as those described in DK 1404/92, EP-A-418187 and EP-A-306466 of Dansk Bioprotein, or air-lift reactors. A loop-type fermentor having static mixers results in a high utilization of the gases (e.g. up to 95%) due to the plug-flow characteristics of the fermentor. Gases are introduced at several positions along the loop and remain in contact with the liquid until they are separated into the headspace at the end of the loop. Continuous fermentation may be achieved using 2-3% biomass (on a dry weight basis) and a dilution rate of 0.02 to 0.50 h⁻¹, e.g. 0.05-0.25 h⁻¹.

Other fermentors may be used in preparing the biomass and these include tubular and stirred tank fermentors.

Typically, the biomass produced from fermentation of natural gas may comprise from 60 to 80% by weight crude protein; from 5 to 20% by weight crude fat; from 3 to 10% by weight ash; from 3 to 15% by weight nucleic acids (RNA and DNA); from 10 to 30 g/kg phosphorus; up to 350 mg/kg iron; and up to 120 mg/kg copper. Particularly preferably, the biomass will comprise from 68 to 73%, e.g. about 70% by weight crude protein; from 9 to 11%, e.g. about 10% by weight crude fat; from 5 to 10%, e.g. about 7% by weight ash; from 8 to 12%, e.g. about 10% by weight nucleic acids (RNA and DNA); from 10 to 25 g/kg phosphorus; up to 310 mg/kg iron; and up to 110 mg/kg copper. The amino acid profile of the protein content can be expected to be nutritionally favourable with a high proportion of the more important amino acids cysteine, methionine, threonine, lysine, tryptophan and arginine. Typically these may be present in amounts of about 0.7%, 3.1%, 5.2%, 7.2%, 2.5% and 6.9%, respectively (expressed as a per cent of the total amount of amino acids). Generally the fatty acids will comprise mainly the saturated palmitic acid (approx. 50%) and the monounsaturated palmitoleic acid (approx. 36%). The mineral content of the product will typically comprise high amounts of phosphorus (about 1.5% by weight), potassium (about 0.8% by weight) and magnesium (about 0.2% by weight).

Typically, the resulting biomass will be produced in the form of a flowable aqueous paste or slurry. Generally this will consist essentially of whole cell material, although a proportion of ruptured cell material may also be present.

The product from the fermentor may be used directly (i.e. without further processing) as, or as a component or precursor to a composition for use as herein described. However, this will generally be subjected to a combination of centrifugation and/or filtration (e.g. ultrafiltration) processes whereby to reduce the water content prior to use. During centrifugation the dry matter content of the biomass is typically increased from about 2 to about 15% by weight, e.g. to about 12% by weight. Ultrafiltration, which may be effected at a temperature of between 40 and 50°C, e.g. between 42 and 46°C, further concentrates the biomass to a product containing from 10 to 30%, preferably from 15 to 25%, e.g. from 15 to 22% by weight biomass. The size exclusion used during ultrafiltration will generally be in the range of about 100,000 Daltons. Following ultrafiltration the biomass may be cooled, preferably to a temperature of from 10 to 30°C, e.g. to about 15°C, for example by passing the concentrated protein slurry from the ultrafiltration unit over a heat exchanger after which it may be held in a buffertank at constant temperature, e.g. for a period of from 1 to 24 hours, preferably 5 to 15 hours, e.g. 5 to 12 hours, at a temperature of from 10 to 20°C, more preferably from 5 to 15°C at a pH in the range of from 5.5 to 6.5.

Following centrifugation and/or ultrafiltration the biomass material will be a relatively viscous protein slurry or paste. Although this may be used directly as or as a component or precursor to a product for use as herein described, this will usually be further processed whereby to remove excess water from the product. The choice of any additional drying step or steps will depend on the water content of the material and the desired moisture content of the final product.

Typically, the product will be further processed in accordance with spray drying techniques well known in the art. Any conventional spray drier with or without fluid bed units may be used, for example the Type 3-SPD spray drier available from APV Anhydro, Denmark. Preferably the inlet temperature for the air in the spray drier may be about 300°C and the outlet temperature may be about 90°C. Preferably the resulting product will have a water content of from about 2 to 10% by weight, preferably from 6 to 8% by weight, e.g. about 5% by weight. The resulting product will typically be a free-flowing granulate having a particle size of from 0.1 to 0.5mm, preferably from 0.15 to 0.2mm.

The immunostimulatory effect of the biomass material herein described renders this suitable for use in enhancing the health and overall vitality of animals (i.e. human and non-human animals). For example, this may be used in preventing diseases or conditions in any animal body which are associated with, caused by, or otherwise contributed to by any exogenous foreign material, for example a pathogenic microorganism. Examples of such diseases and conditions include any disease or infection of viral or bacterial origin, for example gastrointestinal diseases such as salmonellosis, dysentery and common diarrheas (e.g. resulting from infection by microorganisms such as *E*. *coli*, *Enterobacter sp. , Salmonella sp*. and *Proteus sp*.).

One area in which the material finds particular use is as, in or as an additive to an animal feed, especially feed to be consumed by "food" animals, i.e. animals typically raised for human consumption. Examples of "food" animals which may be raised and/or treated according to the methods herein described include pigs, poultry (e.g. chickens), and fish (e.g. salmon, trout, cod, halibut, etc.). The material herein described can be administered to healthy animals to enhance feed utilization and improve the general health and vitality of the animal. For example, this may decrease the dependence of an animal on vaccines and antibiotics.

When used in raising "food" animals, the biomass may be incorporated into conventional animal feeds (e.g. meal, pellets, extruded pellets, meat-based products, cereals, soya-based products, etc.) during production or manufacture in any suitable manner. Alternatively, this may be provided in the form of a feed additive to be mixed with or applied to a conventional animal feed immediately prior to consumption by the animal in an amount sufficient to provide the desired immunostimulatory effect.

The material herein described may also be used to maintain and/or enhance the health and vitality of pets. Accordingly, the material also finds use in the manufacture of a pet food or as a pet food additive.

The term "pet food" as used herein generally refers to any food intended primarily for consumption by pets. Specifically, this includes nutritionally balanced food compositions which are intended to provide substantially the sole diet for the animal. Nutritionally-balanced foods will contain protein, carbohydrates, fats, vitamins and minerals in amounts sufficient for adequate growth and maintenance (i.e. health) of the animal. As used herein the term "pet" is primarily intended to encompass cats and dogs, especially dogs. However, the product herein described may also be used as, in or as an additive to foods intended for consumption by any essentially domesticated or tamed animal or bird, such as rabbits, guinea pigs, tropical fish, birds, etc. The term "pet" is not intended to encompass livestock such as pigs, chickens, cows, etc., or any animal which is primarily bred for human consumption, e.g. fish such as salmon.

The term "pet food additive" as used herein generally refers to any product which is intended to be added to (e.g. incorporated into and/or applied to) a pet food, for example during the manufacturing process or immediately prior to consumption of the food. For example, the biomass material herein described may be dispersed within a pet food or within any component of a pet food (e.g. a gravy or sauce), or coated (either completely or partially) onto an exterior surface of the food. Alternatively, the biomass material may be provided in the form of a pet food additive to be mixed with or applied to a conventional pet food immediately prior to consumption by the animal. For example, this may be liberally sprinkled or sprayed onto the surface of the food in an amount sufficient to provide the required immunostimulatory effects.

Typical pet food compositions to which the biomass material may be added and/or applied include poultry or beef by-products, and soya-based preparations. Preferred compositions are those which are commercially sold and are nutritionally balanced.

The immunostimulatory agent described herein will be employed in animal foods (both pet foods and feed intended for consumption by "food" animals) in an amount effective for this to provide an immunostimulatory effect. Appropriate levels of incorporation of the material will depend on several factors, such as the animal species, age and size of the animal for which this is intended, etc. Suitable levels may readily be determined by those skilled in the art. Typically, when added to conventional animal feeds or pet foods, levels of incorporation in the range of from 1 to 40 wt.%, e.g. from 2 to 20 wt.%, may be used to provide immunostimulatory effects.

The material herein described may also be used to boost the immune system of humans. For example, this may be incorporated into the normal diet as a dietary supplement or may be added to certain foods intended for human consumption in order to provide the desired immunostimulatory effects. Examples of food products which might contain an appropriate amount of the product include, for example, bakery products, meat products, etc. The product may also be used as a meat replacement in vegetarian foods.

When provided in the form of a dietary supplement, the product may be a liquid. However, typically this will be in the form of a powder which can be stored for long periods of time without degradation and which can be added to a food in the appropriate amount or which may be reconstituted into a liquid form (e.g. by the addition of water) immediately prior to consumption. Alternatively, this may be formulated as a composition for enteral (e.g. oral) administration. Suitable compositions may be provided in any orally administrable form, e.g. as capsules or tablets. When provided in the form of a liquid, typically the product will be present in ampoules in which the product is suspended in a suitable liquid medium, e.g. sterile water.

Although primarily described for use as an oral preparation, the immunostimulant herein described may be administered by any other suitable method known in the art, including for example parenteral (e.g. intramuscular, subcutaneous, intraperitoneal or intravenous), rectal or topical administration.

Any composition comprising the product can be administered to a subject (e.g. human or non-human animal) either alone or in combination with at least one physiologically acceptable carrier. As used herein the term "physiologically acceptable carrier" is intended to encompass any substance which can be co-administered with the product and which allows this to perform its intended function. The use of such media are well known in the art. Examples of such carriers include solutions, solvents, dispersion media, delay agents, emulsions and the like.

Suitable dosages which are capable of providing an immunostimulatory effect in humans may be readily determined by those skilled in the art. Appropriate dosages will depend on a number of factors including the age and weight of the patient, and the route of administration. Typical daily dosages for oral administration may lie in the range of from 0.1 to 0.25g, e.g. 0.12 to 0.2g per kg bodyweight.

The products and compositions herein described can also be used in combination with other means of combating infection, for example with vaccines to increase their activity. Thus, the immunostimulatory agents herein described may be associated, either singly or in admixture, with other pharmaceutical products, for example vaccines such as vaccines against yersiniosis or furunculosis.

Viewed from a still further aspect the invention thus provides a composition comprising a biomass material as herein described together with at least one vaccine.

In a still further aspect the invention provides a product containing a biomass material as herein described, and separately a vaccine for simultaneous, separate or sequential use in a method of maintaining and/or enhancing the immune system of a human or non-human animal body.

The invention will now be described in more detail in the following non-limiting Examples, with reference to the accompanying Figures in which:

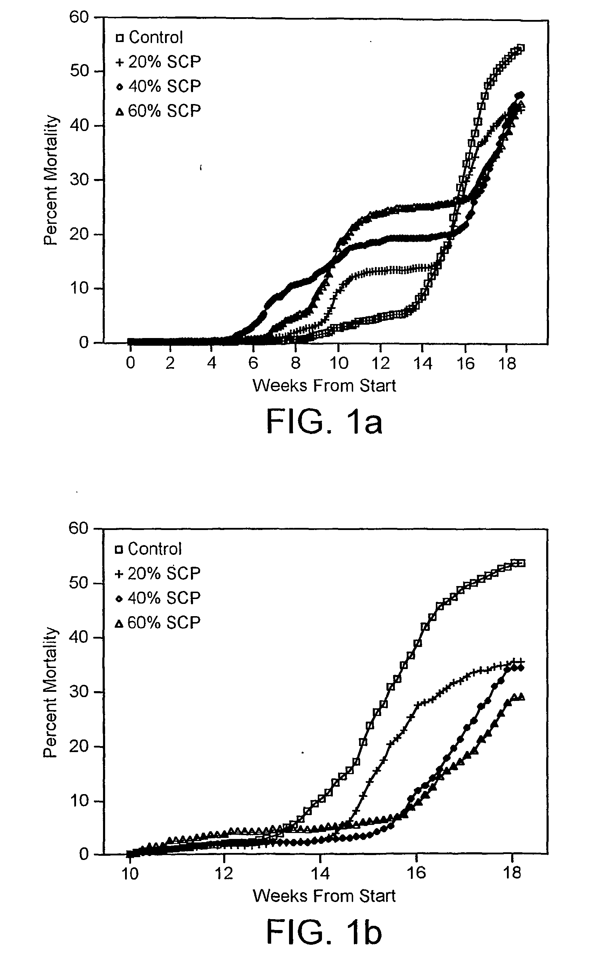

Figure 1a shows accumulated mortality as a percentage of the total number of fish at the start of the study described in Example 2;
Figure 1b shows accumulated mortality given as a percentage of survived fish at week 10 during the last 8 weeks of the study described in Example 2;
Figure 2 shows haemolytic activity of complement in serum from groups of Atlantic salmon fed with a feed containing various doses of a biomass as herein described;
Figure 3 shows lysozyme activity in serum from groups of Atlantic salmon fed with a feed containing various doses of a biomass as herein described;
Figure 4 shows total amount of serum immunoglobulin in groups of Atlantic salmon fed with a feed containing various doses of a biomass as herein described. The results are given as the ratio between the optical density (OD) of the sample and that of a normal serum;
Figure 5 shows antibody responses to *Y. ruckeri,* after vaccination, in serum from groups of Atlantic salmon fed with a feed containing various doses of a biomass as herein described. The results are given as the ratio between the optical density (OD) of the sample and that of a positive reference serum;
Figure 6 shows antibody responses to *A. salmonicida*, after vaccination, in serum from groups of Atlantic salmon fed with a feed containing various doses of a biomass as herein described. The results are given as the ratio between the optical density (OD) of the sample and that of a positive reference serum; and
Figure 7 shows *in vitro* lymphocyte responses to the two mitogens lipopolysaccharide (LPS) from *E*. *coli* and phytohaemaglutinin (PHA) and to two antigenic preparations of *Y. ruckeri* and *A*. *salmonicida* in groups of Atlantic salmon fed with a feed containing various doses of a biomass as herein described. Samples are tested at 8 weeks (Figure 7a) and 18 weeks (Figure 7b) after the start of the trial.
Figure 8 shows the mean titre values (2^{y}) of the Biomass specific antibodies in blood from rats in various dietary groups. For FO, n=8 in each group and for F1, n=5. Standard error is stated for the individual groups.

### Example 1 - Preparation of Biomass

A microbial culture comprising *Methylococcus capsulatus* (Bath) (strain NCIMB 11132), DB3 (strain NCIMB 13287), DB5 (strain NCIMB 13289), and DB4 (strain NCIMB 13288) is produced in a loop-type fermentor by continuous aerobic fermentation of natural gas in an ammonium/mineral salts medium (AMS) at 45°C, pH 6.5. The AMS medium contains the following per litre: 10 mg NH₃, 75 mg H₃PO₄.2H₂O, 380 mg MgSO₄.7H₂O, 100 mg CaCl₂.2H₂O, 200 mg K₂SO₄, 75 mg FeSO₄.7H₂O, 1.0 mg CuSO₄.5H₂O, 0.96 mg ZnSO₄.7H₂O, 120 µg CoCl₂.6H₂O, 48 µg MnCl₂.4H₂O, 36 µg H₃BO₃, 24 µg NiCl₂.6H₂O and 1.20 µg NaMoO₄.2H₂O.

The fermentor is filled with water which has been heat-sterilized at 125°C for 10 secs. Addition of the different nutrients is regulated according to their consumption. With gradual build-up over time, continuous fermentation is operated with 1-3% biomass (on a dry weight basis).

The biomass is subjected to centrifugation in an industrial centrifuge in which the dry matter content is increased from about 2 to 15% by weight. The biomass is further concentrated to a product containing from 15 to 22% by weight biomass using an ultrafiltration unit having an exclusion size of 100,000 Daltons. The temperature during ultrafiltration is maintained between 40 and 50°C. Following ultrafiltration the biomass is cooled, e.g. to a temperature of from 10 to 30°C, by passing the concentrated protein slurry from the ultrafiltration unit over a heat exchanger. The resulting product is a stable culture free from contamination by undesirable bacteria.

Following ultrafiltration, the relatively viscous protein slurry is spray dried. Spray drying at an inlet air temperature of about 300°C and an outlet temperature of about 90°C yields a final product having a water content of about 5% by weight.

The resulting biomass has the following characteristics:

| **Composition (% in product)** | | **Minerals** | |
|---|---|---|---|
| Crude protein* | 66 | Phosphorus | 1.0% |
| Crude fat | 9 | Chlorine | 0.7% |
| Ash | 7 | Sulphur | 0.5% |
| Water | 6 | Calcium | 0.4% |
| Crude fibre | 1 | Potassium | 0.4% |
| N-free extract | 11 | Magnesium | 0.2% |
| **Total** | **100** | Sodium | 0.1% |
| | | Iron | 200 ppm |
| **Amino Acids (% in product)** | | Copper | 90 ppm |
| Lysine | 4.3 | Zinc | 15 ppm |
| Methionine | 1.9 | Arsenic | 0.05 ppm |
| Cystine | 0.4 | Selenium | 0.02 ppm |
| Threonine | 3.1 | Lead | 0.0002 ppm |
| Tryptophan | 1.5 | Cadmium | 0.00002 ppm |
| Leucine | 5.2 | Mercury | <0.02 ppm |
| Isoleucine | 3.2 | | |
| Valine | 4.2 | **Vitamins** | **mg/kg** |
| Tyrosine | 2.8 | Nicotine acid | 123 |
| Phenylalanine | 3.1 | Riboflavin B2 | 69 |
| Histidine | 1.7 | Inositol | 28 |
| Arginine | 4.1 | Thiamin B1 | 11 |
| Alanine | 4.9 | | |
| Aspartic Acid | 6.2 | **Energy** | **MJ/kg** |
| Glutamic Acid | 7.3 | Gross energy | 22.1 |
| Glycine | 3.4 | | |
| Proline | 3.0 | **Other Data** | |
| Serine | 2.5 | Colour | Light brown |
| **Total** | **62.8** | Flavour | Neutral |
| | | Particle Size | 100-300 µm |

| | | | |
|---|---|---|---|
| * on dry weight basis the crude protein content is approx. 70%. | | | |

### Example 2 - Study

The aim of the study was to examine the effect of the biomass produced in Example 1 on the immune system. Unspecific and specific humoral immune responses, cellular immune responses and experimental infection were studied in Atlantic salmon. In the study, which lasted for 18 weeks, groups of fish were given a feed in which the standard protein was replaced by 20%, 40% and 60% biomass. The control group received standard feed.

Unspecific humoral immune responses were measured by lysozyme activity, complement activity and total amount of immunoglobulin in serum.

Development of specific humoral immune responses was studied by measuring antibody levels to *Yersirna ruckeri* and *Aeromonas salmonicida* after vaccination.

Cellular immune responses were studied by the lymphocyte stimulation test in unvaccinated fish.

### MATERIALS AND METHODS

### Fish

**The fish were kept at the research station of** Felleskjøpet, Norsk Bioakva A/S in Dirdal. A total number of 4080 unvaccinated fish of one year old smolts of Atlantic salmon (Salmo salar L.) at an average weight of 50 grams at the start of the trial were split into four groups. Each group was distributed into three parallel tanks of 4m² each and with a total number of 340 fish in each tank. The fish had recently smoltified under natural light conditions. The experiment lasted 18 weeks.

### Water

The tanks were supplied with sea water taken from 90m depth with a salinity of 30 to 33% and an average temperature of 9.0°C (range 7.8 - 11.3°C). Due to problems with the osmoregulation the water salinity was adjusted to 20 - 23% after 8 weeks.

### Feed

The four groups of fish received four different feeds (Tables 1 and 2). Based on a control feed in which fish meal (Norse LT94, Nordsildmel, Bergen) is the main protein source, three test feeds were prepared in which 20, 40 and 60% of the crude protein consisted of the biomass in accordance with Example 1. All protein determinations were done by the Kjeldahl-N*6.25 method and were not corrected for the presence of nucleic acids in the biomass. The amount of carbohydrate from the biomass was balanced with non-heat treated wheat. Feeds with high levels of biomass were supplemented with DL-methionine. The test feeds were produced by means of a lab-pelletizer without the addition of steam at Norsk Bioakva AS, Sandnes, Norway. The amount of fat (lipid) was analyzed by the Soxleths method (Table 2). The amount of carbohydrate including the fibre fraction was determined as the difference between 100% and the remaining components that were measured (Table 2).

Fish were fed by automatic feeders. The tanks were grouped in blocks and each feed was distributed randomly within each block. The daily amount of feed was calculated according to growth tables based on temperature and fish size. Feeding was adjusted every 4 weeks based on actual fish weights.

**Table 1 - Raw material composition in experimental diets as percent of total feed**

| **Feed** | **0% Biomass* Control** | **20% Biomass*** | **40% Biomass*** | **60% Biomass*** |
|---|---|---|---|---|
| Fish meal-LT | 58.2 | 45.7 | 33.2 | 20.7 |
| DL-methionine | 0.0 | 0.0 | 0.01 | 0.06 |
| Wheat | 30.6 | 29.6 | 28.6 | 27.7 |
| Capelin oil | 9.9 | 9.9 | 9.9 | 9.8 |
| Vitamin/mineral premix | 1.3 | 1.3 | 1.3 | 1.3 |
| Biomass acc. Ex. 1 | 0.0 | 13.5 | 27.0 | 40.5 |

| | | | | |
|---|---|---|---|---|
| * as a % of total crude protein in the diet | | | | |

**Table 2 - Chemical composition of experimental diets given as percent of total feed**

| **Feed** | **0% Biomass*** | **20% Biomass*** | **40% Biomass*** | **60% Biomass*** |
|---|---|---|---|---|
| Lipid | 16.3 | 16.7 | 16.9 | 17.0 |
| Protein | 41.9 | 42.3 | 43.2 | 43.6 |
| Carbohydrate | 23.4 | 24.4 | 24.0 | 24.5 |
| Ash | 9.4 | 8.3 | 6.7 | 5.8 |
| Water | 9.0 | 8.3 | 9.2 | 9.1 |

| | | | | |
|---|---|---|---|---|
| * as a % of total crude protein in the diet | | | | |

### Immunization

After 8 weeks on test feed 100 fish from each feed group from one of the three parallel tanks were marked and by intraperitoneal injection immunized with a commercial vaccine against yersiniosis (Ermvaks, Leo vet., Løvens Kemiske fabrik, Denmark). From the same tanks another 100 fish from each group were marked and immunized against furunculosis (Furogen, Aqua Health, Canada).

### Sampling

The fish were blood sampled at the time of vaccination and 4, 8 and 10 weeks after vaccination. Blood was collected from the caudal vein and serum was separated, transported on dry ice and stored at -70°C until further analysis. Tissue from the anterior kidney was sampled at the time of vaccination and 10 weeks after vaccination. The tissues were kept in tissue culture medium on ice for 4 to 6 hours until further analysis.

### Mortality

During the trial mortality occurred during two separate periods of time. The fish were examined by the local veterinarian. During the second period of mortality dead fish were sampled and tested for the presence of infectious pancreatic necrosis virus (IPNV). The whole fish were kept frozen until assaying. Tissue from the pancreas was cut when still frozen and then fixed in phosphate-buffered formalin for 24 h and then embedded into paraffin. The tissue was incubated with a polyclonal rabbit anti-IPNV Sp antiserum. The reaction was developed with an alkaline phosphatase conjugate and Fast red as cromogen.

### Immune parameters

The assays of the various immune parameters were all performed at The National Veterinary Institute, Oslo.

### Complement activity

Complement activity was assayed as spontaneous haemolytic activity (SH) of sheep red blood cells (SRBC). 1% agarose containing 0.25% packed SRBC in 0.094 M sucrose-veronal buffer was mixed at 56°C and poured onto slides coated with agarose. Sample wells were filled with 10 µl of serum samples. The slides were incubated at 22°C for 20h, fixed with formalin, and dried. The diameter of lysed zones were measured. Two-fold dilutions of a salmon serum sample served as a standard. The haemolytic activity was quantified as the percentage of haemolytic activity in the standard.

### Lysozyme activity

Serum lysozyme activity was determined using the *Micrococcus* lysoplate assay. 1% agarose containing *Micrococcus lysodeicticus* in 0.06 M sodium phosphate, pH 6.6, was mixed at 56°C and poured onto slides coated with agarose. Sample wells were filled with 10 µl serum samples diluted 1:10. The slides were incubated at 22°C for 20h, washed in distilled water and dried. The plates were stained with 1.25% methyl violet, then with lugol and destained with ethanol until clear zones appeared. The diameter of lysed zones were measured. Two-fold dilutions of a normal salmon serum served as a standard, and the lysozyme activity was quantified as percentage of lysozyme activity compared to this reference serum.

### Total immunoglobulin

The amount of total immunoglobulin in the serum of non-vaccinated fish from the various feed groups was determined using an Enzyme Linked Immuno Sorbent Assay (ELISA). Polystyrene microtitre plates were coated with a polyclonal rabbit anti-salmon Ig antiserum, at a dilution 1:1000 in coating buffer. Fish sera at the appropriate dilutions were added and incubated at 4°C overnight. The reaction was further developed with a monoclonal anti-salmon Ig antibody and followed by an enzyme labelled sheep anti-mouse Ig conjugate. The results are expressed as the ratio between the optical density (OD) of the test sample and a reference serum.

### Specific antibody responses

The specific antibody responses to *Yersinia ruckeri* and *Aeromonas salmonicida* before and after vaccination were determined using an ELISA in which the plates were coated with a sonicate of either *Y. ruckeri* or *A*. *salmonicida.* The assays were performed as described above. The results are expressed as the ratio between the OD reading of the test sample and a positive reference serum.

### Cellular immune response

The lymphocyte stimulation test was performed in non-vaccinated fish 2 months (at the time of vaccination) and 4 months after start on test feed and was performed as described in the report from the first year of the trial. The following stimulants were used:
1. Lipopolysaccharide (LPS) from *E*. *coli*
2. Phytohaemagglutinin (PHA)
3. Whole, formalin-killed *Y*. *ruckeri*
4. Whole, formalin-killed *A*. *salmonicida*

### Statistical methods

The statistical significance of differences in total immunoglobulin, specific antibody responses and lymphocyte stimulations between various feed groups and between various time points were analyzed using the two-tailed Wilcoxon's rank sum test. The statistical significances of differences in lysozyme and complement activities between various feed groups and time points were analyzed by means of the t-test. The level of significance was for all tests set at p=0.05.

### RESULTS

### Mortality

High mortality was observed during two separate periods in the experiment (Figure la). In the first period, lasting from week 5 to week 11, severe loss of scale, dehydration and skin lesions were observed. The mortality was therefore diagnosed as being caused by defects in the osmoregulation. To avoid further mortality, the salinity of the water was reduced. A reduction in the mortality was immediately observed. From week 14, however, mortality increased again (Figure lb) and the fish showed clinical signs of IPNV infection. After 18 weeks only around 50% of the total amount of the fish was left.

As seen from Table 3 below there were positive reactions for IPNV antigens in fish from 10 out of 12 different tanks. In 6 of the tanks a positive reaction was recorded in all fish sampled. However, there was a tendency to an increase in the number of negative fish by increasing amount of biomass in the feed.

**Table 3 - Presence of IPNV antigens in fish that died during the second outbreak of mortality**

| **Feed** | **Number of positive fish/number of fish tested in different tanks (% positive)** | | | |
|---|---|---|---|---|
| 0% biomass*, control | 17/17 | 15/15 | 15/15 | (100%) |
| 20% biomass* | 18/18 | 0/4 | 15/15 | (89%) |
| 40% biomass* | 13/16 | 0/16 | 15/18 | (56%) |
| 60% biomass* | 6/15 | 15/15 | 1/15 | (49%) |

| | | | | |
|---|---|---|---|---|
| * as a % of total crude protein in the diet | | | | |

It was concluded that IPNV infection was the most prominent cause of the observed mortality during the second period of mortality during the trial.

### Immune parameters

### Complement activity

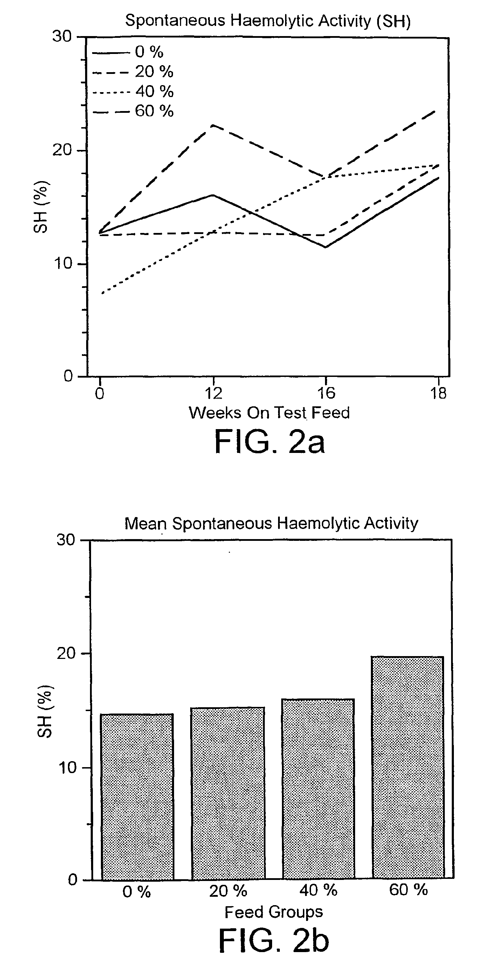

The haemolytic activity increased significantly with increasing time on test feed, while vaccination did not influence the activity (Figure 2). The increase in the amount of biomass in the feed gave a borderline significant increase in activity.

### Lysozyme activity

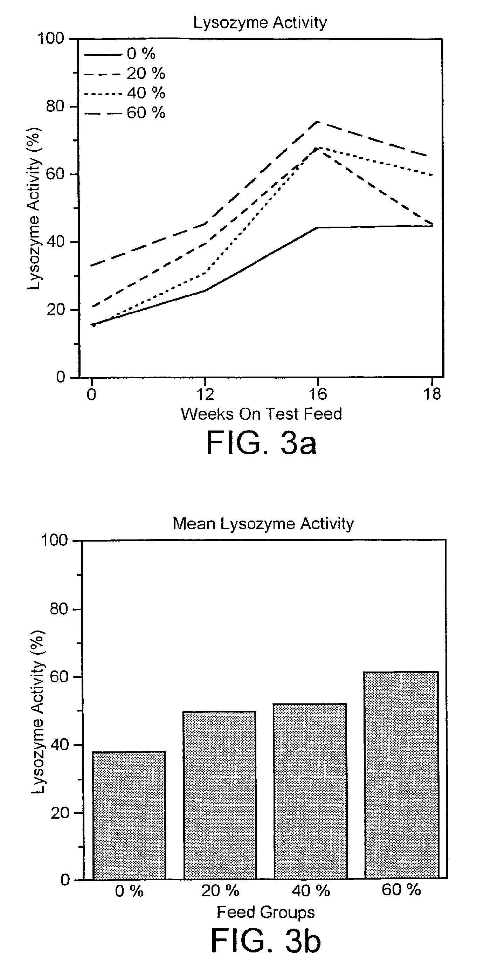

The amount of lysozyme increased significantly both by increasing amount of biomass in the feed and by increasing time on test feed (Figure 3). Vaccination did not influence the amount of lysozyme.

### Total immunoglobulin

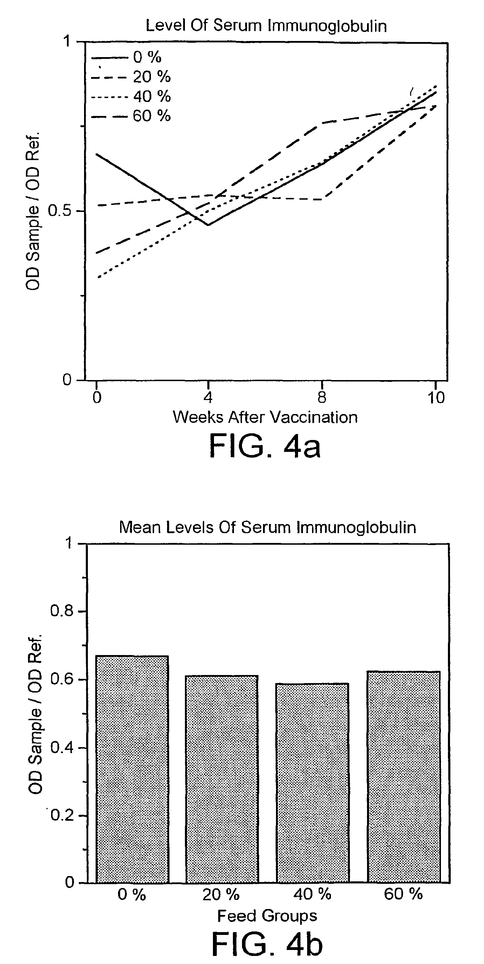

The concentration of total serum immunoglobulin increased significantly by increasing time, but was not influenced by the amount of biomass in the feed (Figure 4). The variations between individual fish within each group were high.

### Specific antibody responses

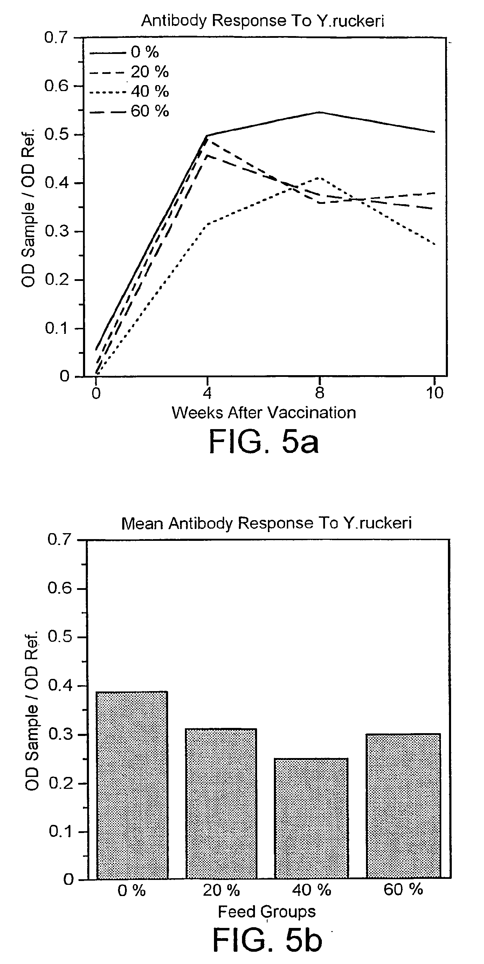

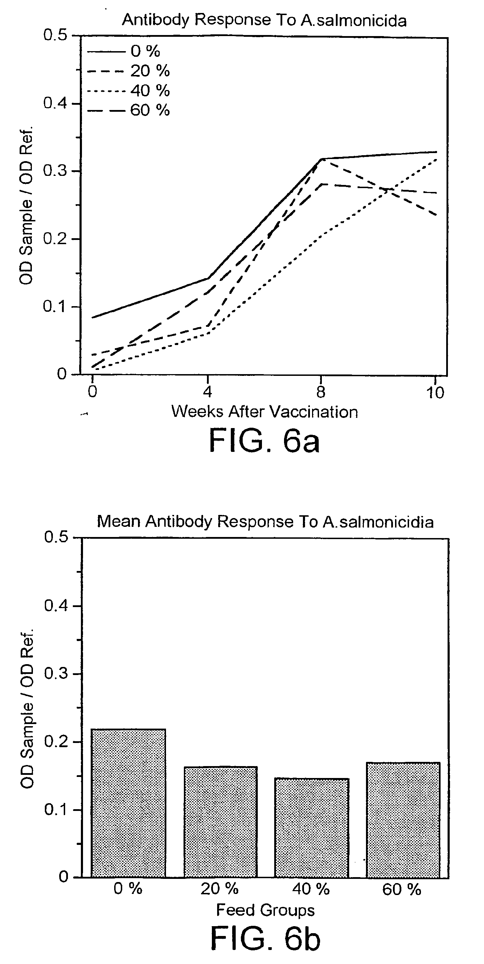

The specific antibody responses to *Y. ruckeri* and *A*. *salmonicida* are shown in Figures 5 and 6, respectively. The responses in the 40% and 60% biomass feed groups were significantly lower than the control group at one or more time points. The variations between individual fish were high, and the antibody responses, in particular to *A*. *salmonicida,* were much lower than the positive reference serum.

### Cellular immune responses

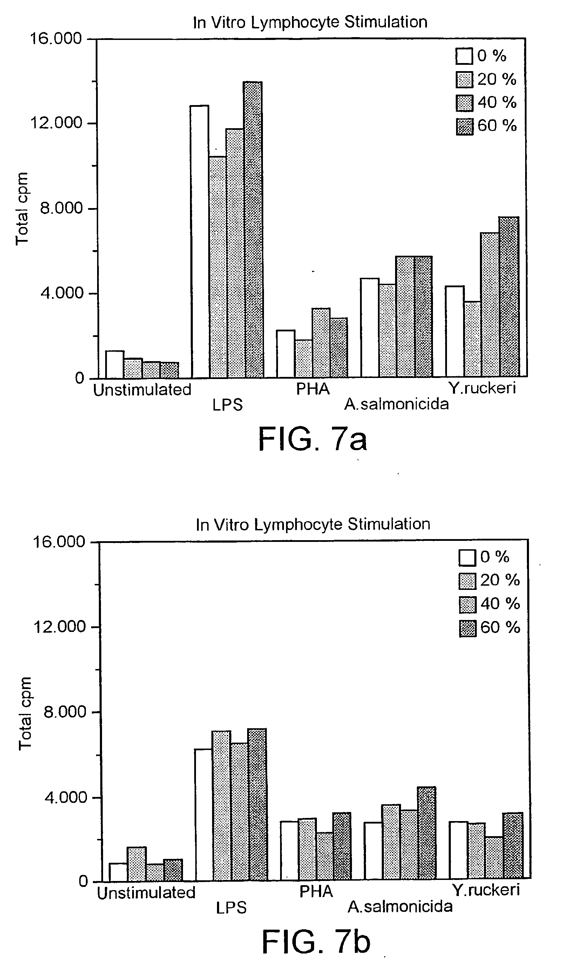

*In vitro* lymphocyte stimulations are given as total counts per minute (cpm) as shown in Figure 7. All groups responded well at 8 weeks after start on test feed, while the responses were somewhat lower after 18 weeks on test feed. There were, however, no statistical differences between the group on control feed and the various test feed groups except for the *Y. ruckeri* stimulated cultures, where there were significantly higher stimulations in the 40% and 60% biomass groups as compared to control feed.

### DISCUSSION

### Mortality

The mortality during weeks 5-11 seemed to be caused by dehydration due to skin lesions. Skin lesions and loss of scales are frequent occurrences in smolts at this stage, and such fish are quite vulnerable to handling.

The second outbreak of mortality was first diagnosed by the veterinarian to be due to IPNV infection. To confirm the diagnosis dead fish were tested for the presence of IPNV antigens, and specific antigens were found in most fish. However, there was a tendency to an increase in the number of fish negative for IPNV by increasing the amount of biomass in the feed. During this outbreak there was also a tendency to reduced mortality with increasing biomass in the diet.

### Immune parameters

The results show an increase in the various unspecific humoral and the cellular immune parameters measured either by increasing time on test feed or by increasing amount of biomass in the diet, or both. It may be that the increase is partly caused by the ongoing infection during part of the test period. It is known that lysozyme activity increases during infections like furunculosis, so it is not unlikely that an infection with IPNV excerts the same type of effect. The level of lysozyme activity found was higher than usually observed and in addition great variations between individual activities were seen, which may indicate that an infection was present. The increase in unspecific immune responses in fish receiving the biomass in accordance with Example 1 is believed to have contributed to the higher resistance to infection shown by these groups during the IPNV outbreak.

Great individual variations were also observed in the results of total serum immunoglobulin and in the specific antibody responses to *Y. ruckeri* and *A*. *salmonicida.* In contrast to previous results there was a tendency to lower responses in the fish receiving 40% and 60% biomass. The level of anti-*A*. *salmonicida* antibody response was in particular much lower than usually seen in smolt vaccinated against furunculosis in all feed groups. Dehydration and the IPNV infection may both have contributed to these results.

### Example 3 - Study

The aim of the study was to analyse rat serum for antibodies specific for the biomass material produced in accordance with Example 1 (herein referred to as "Biomass").

### MATERIALS AND METHODS

### Levels of Biomass specific antibodies were measured in serum from eight rats in each of eight groups in FO: male and female rats fed with 0%, 5.5 wt.%, 11 wt.% and 22 wt.% Biomass in the diet. In addition, the levels in animals from the F1 generation were measured 12 weeks after weaning: five males in each of the four groups

(table 4)

**Table 4 - Diet of the F1 generation and their mothers**

| **Mothers' diet (FO)** | **Pups' diet (F1)** |
|---|---|
| 0% Biomass | 0% Biomass |
| 0% Biomass | 16.5% Biomass |
| 22% Biomass | 0% Biomass |
| 22% Biomass | 16.5% Biomass |

The samples of the F1 generation were selected so that one animal from each litter was tested.

The measurements were carried out using the enzyme-linked immunosorbent assay (Elisa) according to protocol P-01-011. Formulae for the Elisa buffers were as follows:
**Carbonate buffer, pH 9.6**
   14 mM of Na₂CO₃, 35 mM of NaHCO₃
   Storage qualities: 1 month at 4°C
**Incubation buffer, pH 7.4**
   17 mM of Na₂HPO₄
   3.0 mM of NaH₂PO₄
   145 mM of NaCl
   1% triton X-100
   Storage qualities: 2 months at 4°C
**Washing buffer**
   Incubation buffer diluted x 10
**TMP standard solution**
   1.0 g of TMB
   20 ml of CH₃COCH₃
   180 ml of CH₃OH
   Storage qualities: 1 year at -20°0C
**Peroxide standard solution (III)**
   I: 1.47 M of CH₃CO₂H
   II: 1.4 M of CH₃CO₂Na adjusted to pH 5.0 by I
   III: 4.3 g of NaBO₂H₂O₂ + 250 ml of II
   Storage qualities: 6 months in the dark at 4°C
**Peroxide buffer**
   14.3 ml of Peroxide standard solution
   add up to 0.5 1 with distilled water
   Storage qualities: 1 week in the dark at 4°C
**TMB/peroxide solution**
   340 µl of TMB standard solution
   add up to 12 ml with peroxide buffer.
   Storage qualities: None

Filtered extract of the Biomass was diluted 1/68 in carbonate buffer, pH 9.6. Maxi-sorp Elisa microfilter plates (Nunc 442404A, batch 052550) were coated with 100 µl of extract per well and incubated overnight at 4°C.

The plate was washed four times with washing buffer. Double determinations of the serum samples and two standard mixtures of serum from ten animals with an expected high and an expected low level of antibodies specific to the Biomass were put on the plate (100 µl per well) in dilution series from 2⁶ to 2¹³. The plate was incubated on a shaker table (100 rpm) at ambient temperature for one hour after which the plate was washed four times with washing buffer. The plate was then incubated with 100 µl of peroxidase conjugated antibody per well (rabbit-anti-rat-antibodies, Sigma A5795, diluted 1:5000 with incubation buffer) for 1 hour at ambient temperature on a vibrating table (100 rpm). After four washes with washing buffer and one wash with distilled water, 100 µl of TMB/peroxide solution were added per well and the plate was incubated at ambient temperature for 20 minutes. 100 µl of 2 M H₃PO₄ were added to each well, after which the absorbance was measured at 450 nm on a microplate reader (Bio-Tek Instruments, Inc. Elx808).

Raw data was processed using the programme KC4 (Bio-Tek Instruments, Inc.). The raw data was corrected by a blank control and titre values for different specific absorbance values were interpolated. The titre values for the chosen absorption in all analyses were corrected by means of the absorption in the standard mixture. In the few cases in which the titre value was outside the selected absorption, the result was extrapolated by means of the titre curve of the standard mixtures.

### RESULTS

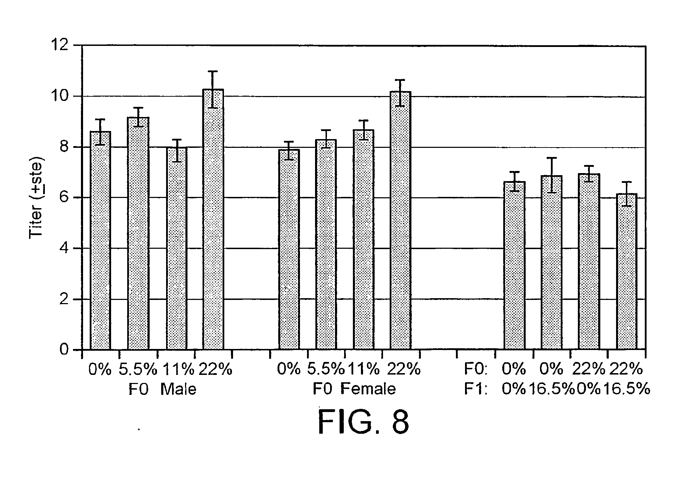

The titre values of the selected groups are presented in attached Figure 8.

In both the males as well as the females of the FO generation, there is a significant difference between the titre value at 0% and 22%, whereas the other groups do not deviate significantly from the control group (α = 0.05). There is no significant difference between the four groups of the F1 generation.

### DISCUSSION

The results show that a level of 22 wt.% Biomass in the animal diets results in an increased level of Biomass specific antibodies. In the next generation, a similar difference is not seen, irrespective of whether the mother generation received Biomass in the diet or not.

### Example 4 - Study

The immunological response to the biomass material of Example 1 (hereinafter referred to as "Biomass") in mice was determined by two methods. The first is an ELISA-assay for measuring the Biomass specific total Ig and IgA in blood and Biomass specific IgA in saliva. The second method is a cell proliferation assay for examination of the ability of the Biomass to stimulate spleen cell proliferation *in vitro.*

In experiments in which the Biomass was administered at different concentrations, a lower dose required more time to induce a response than a higher dose. In experiments in which mice were fed with the Biomass for a period of 14 days and thereafter a normal feed, the level of Biomass specific IgA in the blood was found to drop while a constant level of Biomass specific IgA in saliva was maintained. The local immune response was maintained up to 42 days after the end of feeding with the Biomass even though no systemic response was present.

## Claims

1. A composition for use as a medicament, said composition comprising a biomass obtained from a methanotrophic-bacterium containing culture.

2. A composition for use as a medicament as claimed in claim 1, wherein said biomass is derived from a microbial culture comprising methanotrophic and heterotrophic bacteria.

3. A composition for use as a medicament as claimed in claim 2, wherein said microbial culture comprises *Methylococcus capsulatus* (Bath) (strain NCIMB 11132), DB3 (strain NCIMB 13287) and DB5 (strain NCIMB 13289), optionally in combination with DB4 (strain NCIMB 13288).

4. A composition for use as a medicament as claimed in any one of claims 1 to 3, wherein said microbial culture is produced by fermentation on hydrocarbon fractions or on natural gas, preferably from fermentation on natural gas.

5. A composition as claimed in any one of claims 1 to 4 for use as an immunostimulatory medicament.

6. A composition as claimed in any one of claims 1 to 4 for use in maintaining and/or enhancing the immune system of a human or non-human animal body.

7. A composition as claimed in any one of claims 1 to 4 for use in preventing any disease or condition of viral or bacterial origin.

8. Use of a composition as defined in any one of claims 1 to 4 in the manufacture of a medicament for use as an immunostimulant.

9. Use as claimed in claim 8, wherein said medicament is for use in maintaining and/or enhancing the immune system of a human or non-human animal body.

10. A composition for use as a medicament as claimed in any one of claims 1 to 4, further comprising at least one vaccine.

11. A product containing a biomass as defined in any one of claims 1 to 4, and separately a vaccine, for simultaneous, separate or sequential use in maintaining and/or enhancing the immune system of a human or non-human animal body.

## Patentansprüche

1. Zusammensetzung für die Verwendung als Medikament, wobei die Zusammensetzung eine Biomasse umfaßt, die von einer ein methanotrophes Bakterium enthaltenden Kultur erhalten wurde.

2. Zusammensetzung für die Verwendung als Medikament nach Anspruch 1, wobei die Biomasse von einer mikrobiellen Kultur stammt, die methanotrophe und heterotrophe Bakterien umfaßt.

3. Zusammensetzung für die Verwendung als Medikament nach Anspruch 2, wobei die mikrobielle Kultur Methylococcus capsulatus (Bath) (Stamm NCIMB 11132), DB3 (Stamm NCIMB 13287) und DB5 (Stamm NCIMB 13289), gegebenenfalls in Kombination mit DB4 (Stamm NCIMB 13288), umfaßt.

4. Zusammensetzung für die Verwendung als Medikament nach einem der Ansprüche 1 bis 3, wobei die mikrobielle Kultur durch Fermentation auf Kohlenwasserstoffraktionen oder auf Erdgas, vorzugsweise aus einer Fermentation auf Erdgas erzeugt wird.

5. Zusammensetzung nach einem der Ansprüche 1 bis 4 für die Verwendung als immunstimulierendes Medikament.

6. Zusammensetzung nach einem der Ansprüche 1 bis 4 für die Verwendung bei dem Erhalt und/oder einer Stärkung des Immunsystems eines humanen oder nicht-humanen Tierkörpers.

7. Zusammensetzung nach einem der Ansprüche 1 bis 4 für die Verwendung bei der Verhütung irgendeiner Erkrankung oder eines Zustandes mit viralem oder bakteriellem Ursprung.

8. Verwendung einer Zusammensetzung nach einem der Ansprüche 1 bis 4 bei der Herstellung eines Medikamentes für die Verwendung als Immunstimulans.

9. Verwendung nach Anspruch 8, wobei das Medikament dem Erhalt und/oder der Stärkung des Immunsystems eines humanen oder nicht-humanen Tierkörpers dient.

10. Zusammensetzung für die Verwendung als Medikament nach einem der Ansprüche 1 bis 4, das ferner zumindest einen Impfstoff umfaßt.

11. Produkt, das eine Biomasse nach einem der Ansprüche 1 bis 4 und getrennt davon einen Impfstoff enthält, für die gleichzeitige, getrennte oder aufeinanderfolgende Verwendung beim Erhalt und/oder der Stärkung des Immunsystems eines humanen oder nicht-humanen Tierkörpers.

## Revendications

1. Composition à utiliser comme médicament, ladite composition comprenant une biomasse obtenue à partir d'une culture contenant une bactérie méthanotrophe.

2. Composition à utiliser comme médicament selon la revendication 1, où ladite biomasse provient d'une culture microbienne contenant des bactéries méthanotrophes et hétérotrophes.

3. Composition à utiliser comme médicament selon la revendication 2, où ladite culture microbienne comprend *Methylococcus capsulatus* (Bath) (souche NCIMB 11132), DB3 (souche NCIMB 13287) et DB5 (souche NCIMB 13289), facultativement en combinaison avec DB4 (souche NCIMB 13288).

4. Composition à utiliser comme médicament selon l'une quelconque des revendications 1 à 3, où ladite culture microbienne est produite par fermentation sur des fractions d'hydrocarbure ou sur du gaz naturel, de préférence à partir de fermentation sur du gaz naturel.

5. Composition selon l'une quelconque des revendications 1 à 4 à utiliser comme médicament immunostimulant.

6. Composition selon l'une quelconque des revendications 1 à 4 à utiliser dans la conservation et/ou la stimulation du système immunitaire d'un corps animal humain ou non humain.

7. Composition selon l'une quelconque des revendications 1 à 4 à utiliser dans la prévention de toute maladie ou état pathologique d'origine virale ou bactérienne.

8. Utilisation d'une composition selon l'une quelconque des revendications 1 à 4 dans la préparation d'un médicament à utiliser comme immunostimulant.

9. Utilisation selon la revendication 8, où ledit médicament est à utiliser dans la conservation et/ou la stimulation du système immunitaire d'un corps animal humain ou non humain.

10. Composition à utiliser comme médicament selon l'une quelconque des revendications 1 à 4, comprenant en outre au moins un vaccin.

11. Produit contenant une biomasse selon l'une quelconque des revendications 1 à 4 et séparément un vaccin, à utiliser simultanément, séparément ou séquentiellement dans la conservation et/ou la stimulation du système immunitaire d'un corps animal humain ou non humain.
